# EUROPÄISCHE PATENTSCHRIFT

(11) **EP 1 025 779 B1**
(45) Veröffentlichungstag und Bekanntmachung des Hinweises auf die Patenterteilung: **18.06.2003**
(21) Anmeldenummer: 00101474.5
(22) Anmeldetag: 26.01.2000
(51) Int. Cl.: A47B 88/04, A47B 67/04, F16B 12/02, A47B 47/05

(54) **Lagerschrank insbesondere für Krankenhausbedarf**
Storage cabinet particularly for hospital use
Armoire de stockage notamment pour usage à l'hôpital

(30) Priorität: 03.02.1999 DE 19904247
(43) Veröffentlichungstag der Anmeldung: 09.08.2000
(73) Patentinhaber: Janomed Produktions- und Vertriebsgesellschaft für Krankenhauseinrichtungen mbH, 27793 Wildeshausen (DE)
(72) Erfinder: Janowitz, Bernd, 27793 Wildeshausen (DE)
(74) Vertreter: Eisenführ, Speiser & Partner

(56) Entgegenhaltungen:
- DE-U- 9 215 904
- US-A- 4 882 116
- US-A- 5 472 270

## Beschreibung

Die Erfindung betrifft einen Lagerschrank insbesondere für Krankenhausbedarf (Arzneimittel, Verbrauchsmaterial wie Verbandzeug etc.), mit an beiden Seitenwänden befestigten Rasterschienen, die jeweils eine Reihe von Löchern zur einklinkbaren und lösbaren Halterung von Teleskopschienen für ausziehbare Schubladen aufweisen (wie zum Beispiel aus US5472270 bekannt).

Für die Raumunterteilung von Lagerschränken sind verschiedene Systeme bekannt. Zur Aufbewahrung und Bereithaltung von Krankenhausbedarf wird unterschieden einerseits zwischen Arzneimitteln und andererseits sonstigem Verbrauchsmaterial, zu dem vor allem Verbandszeug, Tupfer, Reinigungsmittel etc. gehören. Arzneimittel werden aus Sicherheitsgründen in verschlossenen Behältern angeliefert und in ortsfest, aber mittels Teleskopschienen ausziehbare Schubladen des Lagerschranks umgefüllt. Hingegen wird bei sonstigem Verbrauchsmaterial so vorgegangen, daß mit seitlichen, schubladenartigen Führungen versehene Behälter, die in entsprechende Führungsschienen des Lagerschranks einschiebbar sind, zwecks Wiederauffüllung dem Lagerschrank entnommen, zur zentralen Versorgungsstelle des Krankenhauses gebracht, dort wieder aufgefüllt, zum Lagerschrank zurückgebracht und in die zugehörigen Seitenführungen des Schrankes geschoben werden.

Im Stand der Technik sind jene Seitenführungen Teile von Trägerwänden, welche innenseitig an den Seitenwänden - ebenso wie die Rasterschienen für die Teleskophalterungen ortsfester Schubladen - durch Schrauben befestigt sind. Damit ist die Verteilung des Innenraumes solcher Lagerschränke einerseits auf ortsfeste Schubladen, andererseits auf entnehmbare Behälter festgelegt. In der Praxis ändert sich jedoch der Bedarf an anteiligem Lagerraum für die beiden Aufbewahrungssysteme, beispielsweise weil die betreffende Station des Krankenhauses einer anderen Abteilung (Chirurgie statt Innere Medizin etc.) zugeordnet wird.

Der Erfindung liegt daher die Aufgabe zugrunde, ein flexibles Lagerschranksystem vorzusehen, bei dem die Verteilung des Schrankinnenraumes auf die beiden Aufbewahrungsarten ohne größeren Aufwand geändert werden kann.

Zur Lösung dieser Aufgabe geht die Erfindung aus von der eingangs geschilderten, beispielsweise aus den DE 88 02 388 U1 und 94 01 876 U1 bekannten lösbaren Anordnung von (horizontalen) Schubladen-Teleskopschienen an (vertikalen) Rasterschienen und zeichnet sich aus durch Trägerwände, die vorderseitig Führungen für einschiebbare Behälter und rückseitig Rastnocken zum Aufklipsen auf die (von Teleskopschienen befreiten) Rasterschienen haben. Erfindungsgemäß werden also alle Seitenwände innenseitig mit Rasterschienen versehen und je nach Bedarf entweder mit Teleskopschienen für Schubladen oder Trägerwänden für entnehmbare Behälter versehen, wobei erstere in bekannter Weise in die Rasterschienen eingeklinkt und letztere in neuartiger Weise auf dieselben Rasterschienen aufgeklipst werden. Durch die Verknüpfung des Befestigens der Trägerwände mit den Rasterschienen der Teleskopschienen wird nicht nur den Rasterschienen eine zweite Nützlichkeit zugewiesen und vor allem die angestrebte Flexibilität erzielt, sondern auch eine entscheidend andere Art der Befestigungskonstruktion vorgesehen, als aus dem DE 92 15 904 U1 für eine Schubladenbox bekannt ist.

Vorzugsweise haben die Löcher in den Rasterschienen und die Rastnocken der Trägerwände einen im wesentlichen quadratischen Umriss. Das erlaubt im Falle der Bestückung mit Teleskopschienen das Einklinken derselben mittels abgekröpfter Ausstanzungen, welche lineare Anlageflächen zum Zusammenwirken mit den Rasterschienen-Löchern haben.

Die Rastnocken bestehen vorteilhaft aus Vertiefungen der aus einer Kunststoffplatte geformten Trägerwand. Sie lassen sich also beim Tiefziehen oder Pressen der Trägerwand unter Einformen der Behälter-Führungen unschwer herstellen. Eine besonders feste Verbindung zwischen der Trägerwand und den Rasterschienen ergibt sich, wenn in Weiterbildung der Erfindung die Rastnocken konisch ausgebildet sind, so daß sie in engen Reibschluß mit den Innenrändern der Rasterschienen-Löcher treten, wenn die Trägerwand auf die Rasterschienen aufgesetzt wird.

Die Zeichnung veranschaulicht die Erfindung an einem Ausführungsbeispiel, darin zeigt:
- Fig. 1: einen teilweisen Längsschnitt durch einen Lagerschrank und somit Draufsicht auf dessen Seitenwand;
- Fig. 2: eine rückwärtige Ansicht der gemäß Fig. 1 in Rasterschienen eingeklinkten Teleskopschiene;
- Fig. 3: eine perspektivische Teilansicht der Teleskopschiene;
- Fig. 4: eine Draufsicht auf eine Trägerwand (mit Führungen für entnehmbare Behälter);
- Fig. 5: eine Seitenansicht der Trägerwand in Fig. 4;
- Fig. 6: einen teilweisen Querschnitt durch einen Lagerschrank mit einer angebrachten und einer anzubringenden Trägerwand;
- Fig. 7: den Ausschnitt "X" (in vergrößertem Maßstab) in Fig. 6;
- Fig. 8: eine Draufsicht auf die in Fig. 6 angebrachte Trägerwand (teilweise weggebrochen) und
- Fig. 9: eine Draufsicht auf die Ausrüstung einer Lagerschrank-Seitenwand teilweise mit einer Trägerwand für die Führung entnehmbarer Behälter und teilweise mit einklinkbaren Teleskopschienen zur ausziehbaren Halterung von Schubladen.

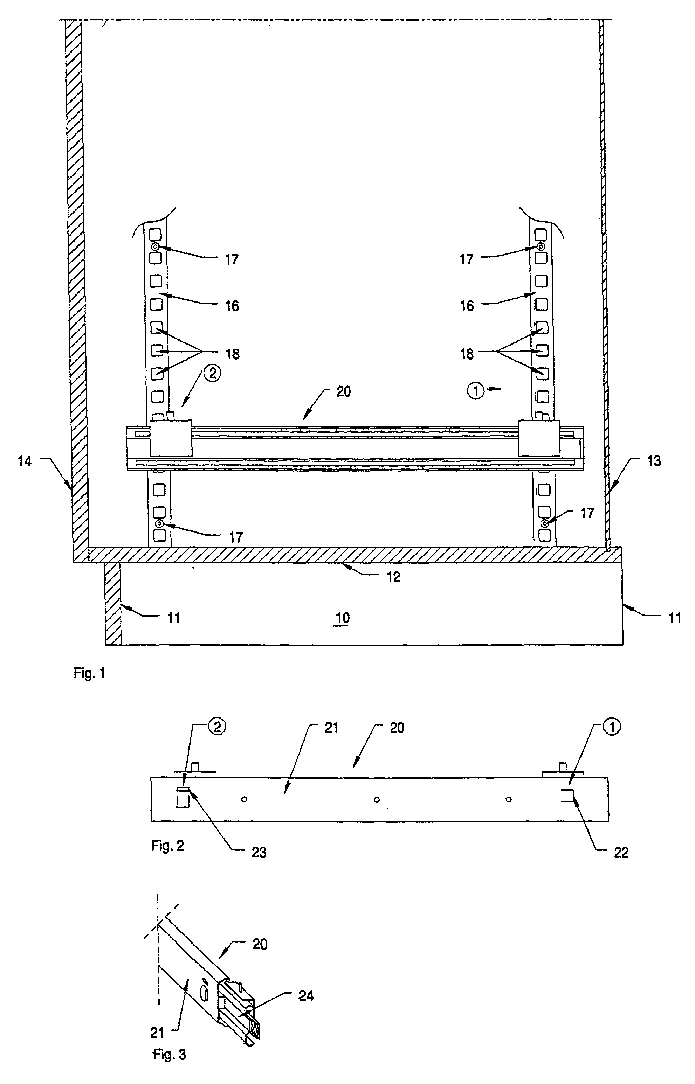

Der in Fig. 1 in teilweisem Längsschnitt gezeigte Lagerschrank besitzt einen Sockel 10 mit Sockelblende 11, einen Boden 12, eine Rückwand 13 und eine Klapptür 14. Von den beiden sich zwischen der Rückwand 13 und der Tür 14 erstreckenden Seitenwänden ist die eine Seitenwand 15 in Draufsicht sichtbar. An ihr sind - vorn und hinten - vertikale Rasterschienen 16 mittels Schrauben 17 befestigt. Die Rasterschienen 16 haben in bekannter Weise einen flach O-förmigen Querschnitt, so daß ihr in Fig. 1 sichtbarer Steg Abstand von der inneren Oberfläche der Seitenwand 15 besitzt. In diesem Steg der Rasterschiene 16 ist in ebenfalls bekannter Weise eine Reihe von Löchern 18 eingestanzt, die gleichen Abstand voneinander haben und vorliegend einen quadratischen Umriss besitzen.

An den Rasterschienen 16 lassen sich im ganzen mit 20 bezeichnete Teleskopschienen (nur eine ist in Fig. 1 dargestellt) dadurch befestigen, dass ihr Trägerprofil 21 mit ausgestanzten und abgekröpften Haken 22 bzw. 23 versehen ist. In der aus dem DE 88 02 388 U1 bekannten Weise wird der nach hinten offene Haken 22 in eine Öffnung 18 der hinteren (der Rückwand 13 benachbarten) Rasterschiene 16 eingeschoben, worauf der nach unten offene Haken 23 in das entsprechende Loch 17 der vorderen Rasterschiene 16 eingehängt werden kann. Damit ist das Tragprofil 21 an der Seitenwand 15 befestigt, und eine - nicht dargestellte - Schublade kann am ausziehbaren Profil 24 der Teleskopschiene 20 befestigt werden. Bis hierhin gehört das System zum Stand der Technik.

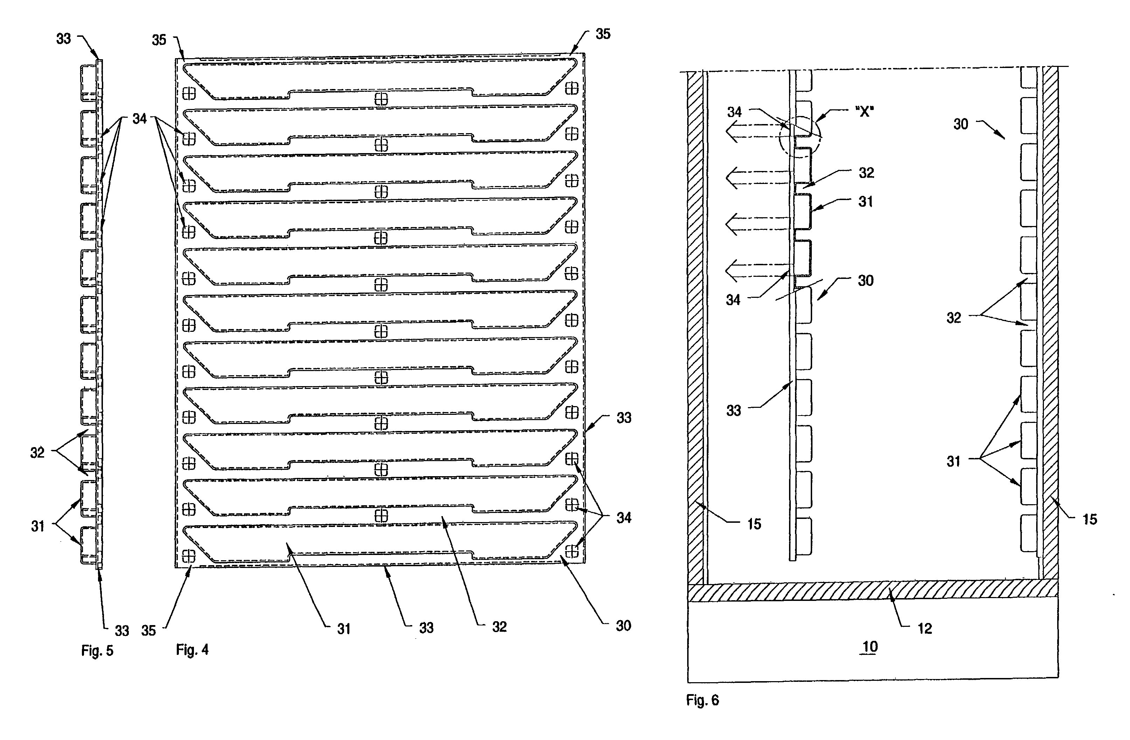

Fig. 4 zeigt in Draufsicht eine - in ihrer Grundausbildung ebenfalls bekannte - Trägerwand 30. Sie besteht aus einer Kunststoffplatte mit im wesentlichen trapezförmig geformten Vorsprüngen 31, die zwischen sich Führungsschienen 32 zur verschiebbaren Führung entnehmbarer - nicht dargestellter - Behälter bilden. Der Rand 33 der Trägerwand 30 ist umlaufend abgekröpft.

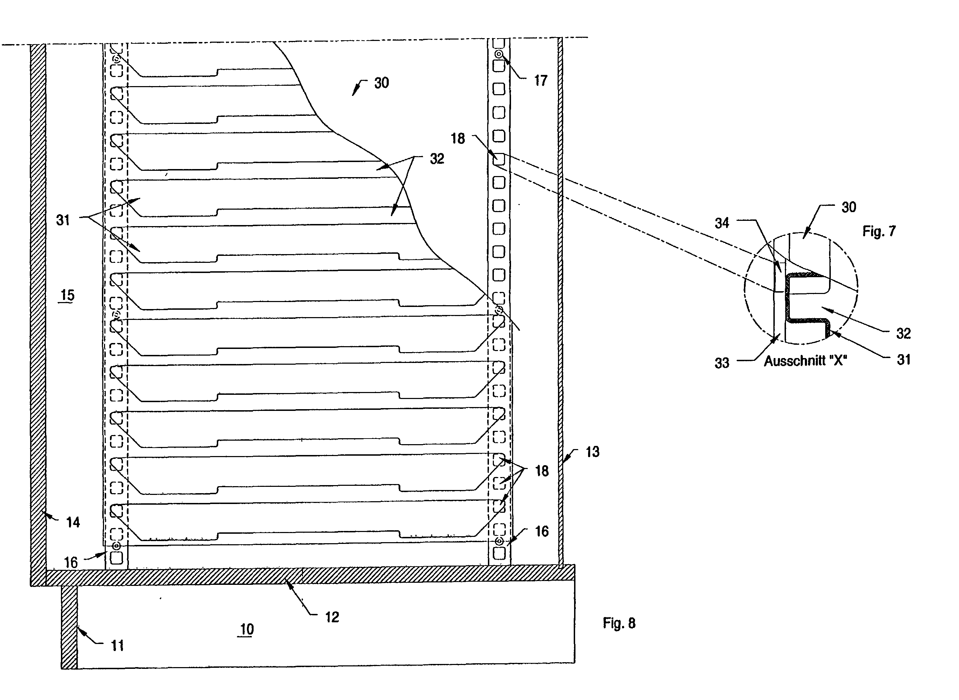

Aus der Grundebene der die Trägerwand 30 bildenden (Kunststoff-)Platte, welche der Grundebene der Führungsschienen 32 entspricht, sind erfindungsgemäß nach der den Vorsprüngen 31 entgegengesetzten Seite Vertiefungen 34 ausgebildet, deren Form und Anordnung insbesondere aus der vergrößerten Darstellung des Ausschnitts "X" (aus Fig. 6) in Fig. 7 hervorgeht. Die Fig. 6 und 8 - letztere in Verbindung mit Fig. 7 - veranschaulichen, wie die Trägerwand 30 an den Rasterschienen 16 befestigt werden kann, indem die - leicht konisch ausgebildeten - Vertiefungen 34 in die Rasterlöcher 18 eingeführt werden. Damit der abgekröpfte Rand 33 die etwas erhabenen Rasterschienen 16 durchläßt, sind im Rand 33 Aussparungen 35 vorgesehen.

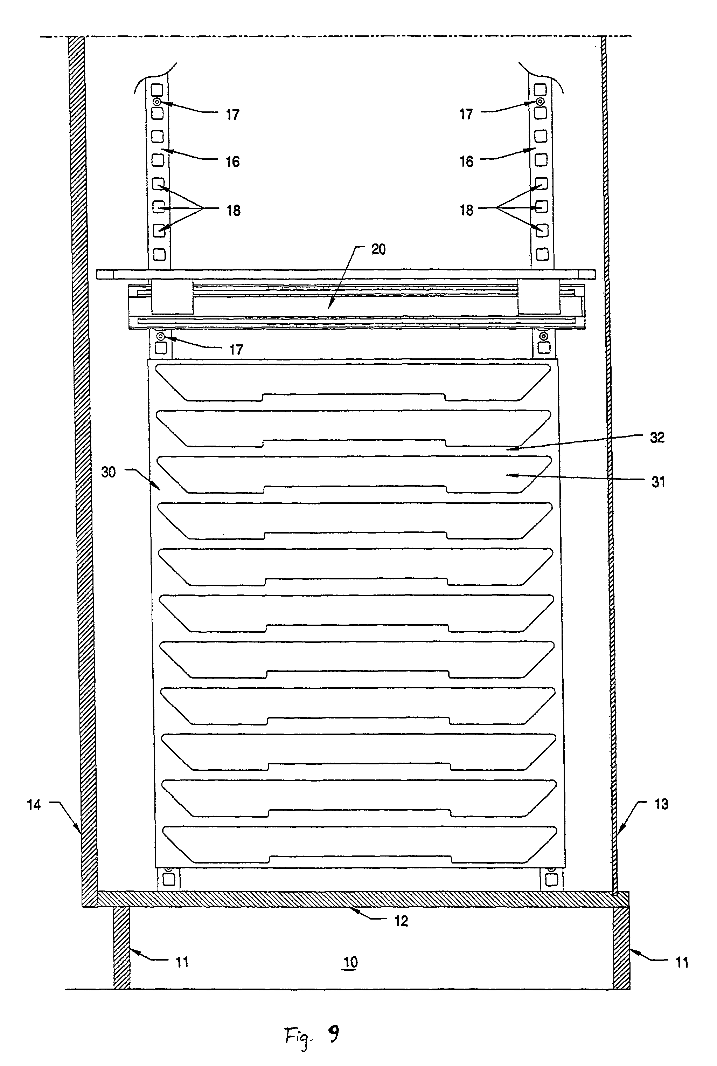

Fig. 9 macht deutlich, daß es auch möglich ist, nicht nur nebeneinander liegende Teilräume eines Lagerschrankes unterschiedlich auszurüsten, sondern über die Höhe ein und desselben Schrankes teilweise Schubladen mit Teleskopführungen und teilweise entnehmbare Behälter mit entsprechenden Trägerwänden aufzunehmen.

## Patentansprüche

1. Lagerschrank insbesondere für Krankenhausbedarf (Arzneimittel, Verbrauchsmaterial wie Verbandszeug etc.), mit an beiden Seitenwänden (15) befestigten Rasterschienen (16), die jeweils eine Reihe von Löchern (18) zur einklinkbaren und lösbaren Halterung von Teleskopschienen (20) für ausziehbare Schubladen aufweisen,
**gekennzeichnet durch** Trägerwände (30), die vorderseitig Führungen (32) für einschiebbare Behälter und rückseitig Rastnocken, vorteilhaft Vertiefungen (34), zum Aufklipsen auf die von Teleskopschienen befreiten Rasterschienen (16) haben.

2. Lagerschrank nach Anspruch 1,
**dadurch gekennzeichnet, daß** die Löcher (18) in den Rasterschienen (16) und die Rastnocken (34) der Trägerwände (30) einen im wesentlichen quadratischen Umriß haben.

3. Lagerschrank nach Anspruch 1 oder 2,
**dadurch gekennzeichnet, daß** die Rastnocken aus Vertiefungen (34) der aus einer Kunststoffplatte geformten Trägerwand (30) bestehen.

4. Lagerschrank nach einem der vorhergehenden Ansprüche,
**dadurch gekennzeichnet, daß** die Rastnocken (34) konisch ausgebildet sind.

## Claims

1. Storage cabinet, in particular for hospital supplies (medicines, materials such as dressings and bandages, etc.), with perforated rails (16) which are secured on both side walls (15) and which each have a series of holes (18) for snap-in and releasable mounting of telescopic rails (20) for pull-out drawers, **characterized by** support walls (30) which, at the front, have guides (32) for insertable containers and, at the back, have latching elements, advantageously recesses (34), for clipping onto the perforated rails (16) freed from telescopic rails.

2. Storage cabinet according to Claim 1, **characterized in that** the holes (18) in the perforated rails (16) and the latching elements (34) of the support walls (30) have a substantially square contour.

3. Storage cabinet according to Claim 1 or 2, **characterized in that** the latching elements consist of recesses (34) in the support wall (30) formed from a plastic panel.

4. Storage cabinet according to one of the preceding claims, **characterized in that** the latching elements (34) are of conical configuration.

## Revendications

1. Armoire de stockage, notamment pour usage à l'hôpital (médicaments, consommables comme bandages, etc.) avec des glissières tramées (16) fixées aux deux parois latérales (15) et présentant respectivement une rangée de trous (18) pour la fixation encliquetable et détachable de glissières télescopiques (20) pour des tiroirs coulissants, **caractérisée par** des parois porteuses (20) qui présentent à l'avant des guides (32) pour des récipients rétractables et à l'arrière des ergots d'arrêt, de façon avantageuse des renfoncements (34), à accrocher sur les glissières tramées (16) libérées des glissières télescopiques.

2. Armoire de stockage selon la revendication 1, **caractérisée en ce que** les trous (18) dans les glissières tramées (16) et les ergots d'arrêt (34) des parois porteuses (30) présentent un contour substantiellement carré.

3. Armoire de stockage selon la revendication 1 ou 2, **caractérisée en ce que** les ergots d'arrêt se composent de renfoncements (34) de la paroi porteuse (30) formée à partir d'une plaque de matière plastique.

4. Armoire de stockage selon l'une quelconque des revendications précédentes, **caractérisée en ce que** les ergots d'arrêt (34) sont réalisés de forme conique.
